# EUROPÄISCHE PATENTSCHRIFT

(11) **EP 0 711 567 B1**
(45) Veröffentlichungstag und Bekanntmachung des Hinweises auf die Patenterteilung: **26.02.2003**
(21) Anmeldenummer: 95117467.1
(22) Anmeldetag: 07.11.1995
(51) Int. Cl.: A61L 17/00

(54) **Chirurgisches Nahtmaterial und seine Verwendung in der Chirurgie**
Surgical suture material and its use in surgery
Suture chirurgicale et son utilisation en chirurgie

(30) Priorität: 10.11.1994 DE 4440095
(43) Veröffentlichungstag der Anmeldung: 15.05.1996
(73) Patentinhaber: Aesculap AG & Co. KG, 78532 Tuttlingen (DE)
(72) Erfinder: Goldmann, Helmut Dr., D-78532 Tuttlingen (DE); Hierlemann, Helmut Dr., D-73033 Göppingen (DE); Müller, Erhard Dr., D-70565 Stuttgart (DE); Planck, Heinrich Dr., D-72622 Nürtingen (DE)
(74) Vertreter: Patentanwälte Ruff, Wilhelm, Beier, Dauster & Partner

(56) Entgegenhaltungen:
- EP-A- 0 608 139
- BE-A- 872 208
- DE-A- 2 206 144
- GB-A- 2 033 411

## Beschreibung

Die vorliegende Erfindung betrifft chirurgisches Nahtmaterial aus resorbierbarem synthetischem Polymer und seine Verwendung in der Chirurgie.

Für den Wundverschluß verwendbares chirurgisches Nahtmaterial umfaßt nichtresorbierbare und resorbierbare Materialien. Bekannt sind resorbierbare chirurgische Nähfäden auf Basis von natürlichen biologischen Materialien, insbesondere Catgut und resorbierbare synthetische Nähfäden. Resorbierbares synthetisches Nahtmaterial kann unter anderem aus Polyglykolsäure (PGA) hergestellt werden. Die Nähfäden erfahren in physiologischer Umgebung eine Hydrolyse. Als Maß für den hydrolytischen Abbau des Polymermaterials dient der Reißkraftverlust von 50 %, der auch als Halbwertszeit bezeichnet.wird. Aus geflochtenen PGA-Multifilamenten gebildete chirurgische Nähfäden (z. B. unter dem Warenzeichen Dexon erhältlich) zeigen innerhalb von 21 Tagen 50 %igen Reißkraftverlust und eine Resorption der Hydrolysate innerhalb von 100 bis 120 Tagen. Ähnliche Eigenschaften weist ein aus Glykolid-Lactid-Copolymer mit einem Comonomerenverhältnis von 90 : 10 hergestellter multifiler Nähfaden auf (unter dem Warenzeichen Vicryl erhältlich). Er verliert in vivo nach 25 Tagen 50 % seiner Ausgangsfestigkeit und ist nach mehr als 80 Tagen resorbiert.

Bisher bekanntes resorbierbares synthetisches Nahtmaterial in Form von multifilen Nähfäden weist eine Reihe von Nachteilen auf wie beispielsweise schlechter Gewebedurchzug, Sägewirkung oder Kapillarität. Monofilamente aus den beschriebenen Polymeren weisen eine hohe Steifigkeit auf und zeigen ein schlechtes Knüpfverhalten, was für ihre Verwendung als chirurgisches Nahtmaterial nachteilig ist.

Es sind auch monofile Nähfäden auf Basis von Polyglykolsäure bekannt, die weichmachende Komponenten in der PGA-Matrix enthalten. Aus einem Copolymerisat aus Glykolid und Trimethylencarbonat im Verhältnis 68 zu 32 (unter Warenzeichen Maxon erhältlich) kann ein chirurgischer monofiler Faden hergestellt werden, dessen Festigkeit bis zu 50 % der Ausgangsfestigkeit über ca. 5 Wochen erhalten bleibt und der nach ca. 7 Monaten vollständig resorbiert ist. Flexible, brauchbare Monofile können ebenfalls aus Polydioxanon (unter Warenzeichen PDS erhältlich) extrudiert werden. Reißkraftverlust und Resorptionszeit sind mit Fäden aus Glykolid/Trimethylencarbonat (Warenzeichen Maxon) vergleichbar (siehe folgende Tabelle).

| Handelsübliche chirurgische Nähfäden | | | | |
|---|---|---|---|---|
| Polymer | Handelsname | Faden | 50 % Reißkraftverlust (Wochen) | Resorptionszeit (Monate) |
| Polyglykolsäure | Dexon^{R} | geflochten | 3* | 3-4* |
| Glykolid/Lactid-Copolymer (90/10) | Vicryl^{R} | geflochten | 3-4* | 3* |
| Glykolid/TMC-Copolymer (68/32) | Maxon^{R} | monofil | 5** | 7** |
| Polydioxanon | PDS^{R} | monofil | 5*** | 6*** |

| | | | | |
|---|---|---|---|---|
| * P.F. Nockermann: Die chirurgische Naht, Georg Thieme Verlag Stuttgart, 3. Aufl., 1980, S. 42 | | | | |
| ** A. R. Katz, D. P. Mukberjec, A. L. Kaganov und S. Gordon in: Surg. Gynecol. Obstet. 161, 213 (1985) | | | | |
| *** J. A. Ray, N. Doddi, D. Regula, J. A. Williams und A. Melveger in: Surg. Gynecol. Obstet. 153, 497 (1981) | | | | |

Diese monofilen Fäden zeigen jedoch ein sehr langsames Abbauverhalten in lebendem Gewebe und sind erst nach mehreren Monaten vollständig vom Körper resorbiert. Im Vergleich zu bekannten multifilen Fäden ist ihre Resorptionszeit doppelt so lang. Neuere Entwicklungen umfassen daher monofile Nähfäden aus resorbierbaren Polymeren mit kürzeren Halbwertszeiten und schnellerer Resorption.

US 4653497 beschreibt die zweistufige Herstellung von kristallinen Copolymeren aus 1,4-Dioxanon und Glykolid sowie die Eigenschaften von daraus hergestellten monofilen Fäden. Diese Monofile weisen in vivo nach 4 Wochen noch 10 bis 30 % ihrer Festigkeit auf und sind nach ca. 4 bis 5 Monaten resorbiert.

In US 4700704 sind Copolymere aus Glykolid und ∈-Caprolacton und daraus hergestellte Monofilamente beansprucht. Diese chirurgischen monofilen Nähfäden zeigen bei Implantation nach 7 Tagen 44 bis 62 % ihrer Festigkeit und nach 14 Tagen 11 bis 37 % Festigkeitserhalt.

Entsprechend US 4441496 können Copolymere bestehend aus 1,4-Dioxanon und 2,5-Morpholinodionen zu Monofilamenten verarbeitet werden, deren Festigkeit nach 28 Tagen bis zu 50 % vom Ausgangswert beträgt und die nach 90 bis 180 Tagen vollständig resorbiert sind.

DE-OS 2206144 offenbart Copolymere aus Glykolid als Hauptkomponente und anderen cyclischen Comonomeren wie beispielsweise Ethylencarbonat und Trimethylencarbonat. Bei einem Anteil von 15 bis 25 % Ethylencarbonat beträgt die Absorptionszeit 30 bis 60 Tage (bei 15 %) bzw. 15 bis 30 Tage beim höheren Ethylencarbonatgehalt.

US 4243775 und US 4300565 beschreiben die aufeinanderfolgende Polymerisation von Glykolid und Trimethylencarbonat, die zu Blockpolymeren führt. Aus diesem sequentiellen Copolymer hergestellte monofile Fäden haben bei einem Trimethylencarbonatgehalt von 15 Gew.-% nach 21 Tagen in vivo noch eine Linearreißfestigkeit von 45 % des Ausgangswertes, bei einem Gewichtsanteil von 45 % nach 21 Tagen noch eine Linearreißkraft von 38 %.

In EP-A-0 608 139 sind Medizinprodukte beansprucht, die aus einem bioabsorbierbaren Elastomer hergestellt sind. Hierbei handelt es sich um eine statistisches Copolymer aus
a) 30 bis 50 Gew.% ε-Caprolacton, Trimethylencarbonat (TMC), einem Etherketon oder Mischungen davon, und
b) einem sich ergebenden Anteil Glycolid, Paradioxanon oder Mischungen davon.
In Beispiel 11 wird ein Copolymer beschrieben bestehend aus 70% Glykolid und 30% TMC. Auch diese Copolymer ist ein Elastomer.

In GB 2033411 werden ebenfalls sequentielle Copolymere aus Glykolid und Trimethylencarbonat (TMC) beansprucht, mit einem bevorzugten TMC-Gehalt von 10 bis 20 %. Die verbleibende Festigkeit extrudierter, verstreckter Monofile beträgt nach 21 Tagen der Implantation noch 45 %.

In einigen Bereichen des chirurgischen Wundverschlusses sind lange Resorptionszeiten und ein langer Erhalt der Reißkraft unnötig, da das Gewebe seine Festigkeit schnell wiedergewinnt und der Faden damit seine Funktion verliert. Beispiele solcher Indikationen sind Nähte im Bereich des Darmes, im Bereich der Schleimhaut des Zahn-Mund-Kiefer-Bereiches und gynäkologische Anwendungen. Bei chirurgischen Nähfäden für diese Verwendungen ist ein beschleunigter hydrolytischer Abbau und damit eine schnellere Resorption erwünscht. Es genügt, wenn eine ausreichende Nahtfestigkeit für einige Tage besteht. Eine gute Knotenreißfestigkeit und gute Knüpfbarkeit ist dabei für eine sichere und zuverlässige Anwendung erforderlich. Derzeit sind für diese Indikationen aus synthetischem Material nur multifile Nähfäden erhältlich, deren bekannte Nachteile oben genannt wurden.

Es stellte sich daher die Aufgabe, ein chirurgisches Nahtmaterial aus resorbierbarem synthetischem Polymer in Form eines monofilen Filaments mit beschleunigtem Abbauverhalten und verbesserter Resorption zur Verfügung zu stellen, das die Nachteile bekannten Nahtmaterials überwindet, einfach und kostengünstig herzustellen sowie einfach und zuverlässig anzuwenden ist.

Diese Aufgabe wird gelöst durch ein chirurgisches Nahtmaterial aus resorbierbarem synthetischen Polymer, das dadurch gekennzeichnet ist, daß es aus Glykolid-Trimethylencarbonat-Copolymer mit einem Glykolid-Gehalt größer als 76 Gew.-% mit statistischer Verteilung der Monomeren gebildet ist. Vorzugsweise kann das erfindungsgemäße Nahtmaterial als monofiles Filament mit einem Durchmesser von 0,001 bis 0,8 mm ausgebildet sein. In einem solchen erfindungsgemäßen Material kann der Glykolid-Gehalt, bezogen auf das gesamte Copolymer vorzugsweise mehr als 78 Gew.-%, betragen. Insbesondere kann sein Glykolid-Gehalt 80 bis 99 Gew.-%, bezogen auf das gesamte Copolymer, betragen. In einer bevorzugten Ausführungsform der Erfindung kann sein Glykolid-Gehalt 83 bis 93 Gew.-%, bezogen auf das gesamte Copolymer, betragen.

Überraschenderweise wurde festgestellt, daß aus statistischen Copolymeren aus Glykolid und Trimethylencarbonat (TMC) mit einem Glykolid-Gehalt von mehr als 76 Gew.-% Monofilamente hergestellt werden können, die für chirurgisches Nahtmaterial erforderliche sehr gute Eigenschaften aufweisen, insbesondere bezüglich physikalischer Eigenschaften und praktischer Handhabung, und die wesentlich schneller degradieren und resorbiert werden als die bisher im Stand der Technik bekannten Monofile aus denselben Comonomeren, die sequentiell polymerisiert, d. h. zu Block-Copolymeren, umgesetzt wurden.

Nahtmaterial aus statistischem Copolymer gemäß der vorliegenden Erfindung zeichnet sich mit Vorteil durch eine beschleunigte Resorbierbarkeit in lebendem Gewebe aus. Vorzugsweise kann seine Resorbierbarkeit weniger als 100 Tage betragen. Insbesondere kann seine Resorptionszeit in vivo 35 bis 91 Tage betragen. Bevorzugt kann seine Resorptionszeit in vivo 42 bis 56 Tage betragen.

Der Abbau des erfindungsgemäßen Polymers erfolgt im Körper eines Tieres oder eines Menschen durch Stoffwechselvorgänge. An der Umsetzung sind Körper- und Gewebeflüssigkeiten beteiligt. Durch Hydrolyse wird die Polymerkette in kleinere und leichter lösliche Fragmente gespalten. Die Bruchstücke werden ggf. unter Beteiligung enzymatischer Prozesse weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Nahtmaterials beim Patienten ist es wichtig, daß sich während des Abbauvorganges keine schädlichen Metaboliten bilden oder anreichern. Polyglykolsäure zeichnet sich insbesondere dadurch aus, daß bei ihrer Zersetzung in vivo keine toxischen Zerfallsprodukte gebildet werden. Das erfindungsgemäß als Comonomer verwendete Trimethylencarbonat ist ebenfalls durch gute Verträglichkeit und Vermeidung toxischer Reaktionen gekennzeichnet.

Das statistische Glykolid-TMC-Copolymer gemäß der vorliegenden Erfindung unterscheidet sich von den bisher zur Herstellung von chirurgischem Nahtmaterial üblichen sequentiellen Glykolid-TMC-Copolymeren durch die modifizierte Abfolge der Monomereinheiten in der Makromolekülkette. Dies beeinflußt auch die Wechselwirkungen zwischen den einzelnen Kettenmolekülen in einem gebildeten Filament. Wie den Fachleuten auf dem Gebiet der Fasertechnologie bekannt ist, hängen die physikalischen und mechanischen Eigenschaften einer Faser von der Orientierung und Struktur der Kettenmoleküle ab, insbesondere der Ausbildung amorpher und kristalliner Bereiche. Wie Analysen der Mikrostruktur ergeben kann das erfindungsgemäße Nahtmaterial aus statistischem Glykolid-TMC-Copolymer eine Kristallinität von 15 bis 40 % aufweisen. Es kann ferner gekennzeichnet sein durch eine inhärente Viskosität (Grenzviskosität) von 1,0 bis 2,0 dl/g, insbesondere von 1,2 bis 1,6 dl/g, gemessen in 0,1 % Hexafluorisopropanol (HFIP) bei 30 °C.

Mit Vorteil zeichnet sich das erfindungsgemäße Material durch seine mechanischen Eigenschaften aus. So kann das monofile Nahtmaterial gemäß der Erfindung eine Zugfestigkeit von 250 bis 900 N/mm², insbesondere von 300 bis 650 N/mm² aufweisen. Diese Zugfestigkeitswerte entsprechen den Zugfestigkeiten, die für anerkannt gute resorbierbare chirurgische Nähfäden aus dem Stand der Technik angegeben und von den Pharmakopöen gefordert werden, oder überschreiten diese sogar noch deutlich. Das erfindungsgemäße chirurgische Nahtmaterial besitzt eine Bruchdehnung von 25 bis 45 %, insbesondere von 30 bis 40 %. Auf diese Weise ist das Monofilament gemäß der Erfindung für den Einsatz bei chirurgischen Anwendungen besonders geeignet.

Der Abbau der Polymerketten während der Resorption geht einher mit einer Verringerung der mechanischen Festigkeit des Filamentmaterials. Nach 3 bis 10 Tagen, insbesondere nach 4 bis 6 Tagen kann das erfindungsgemäße chirurgische Nahtmaterial noch 50 % seiner Zugfestigkeit aufweisen. Die Degradation des Nahtmaterials führt dazu, daß es in vivo innerhalb von 7 bis 28 Tagen einen vollständigen Reißkraftverlust aufweisen kann. Insbesondere kann ein erfindungsgemäßes Material in vivo innerhalb von 9 bis 21 Tagen einen vollständigen Reißkraftverlust aufweisen.

Untersuchungen des Verhaltens der mechanischen Eigenschaften im Laufe der Zeit und damit des Abbauverhaltens des erfindungsgemäßen Nahtmaterials zeigen deutliche Unterschiede zum Verhalten bekannter resorbierbarer chirurgischer Nahtmaterialien aus dem Stand der Technik. Die Untersuchungen werden in den nachfolgenden Beispielen und in den begleitenden Figuren noch ausführlicher beschrieben. Wie oben schon erwähnt zeichnen sich die chirurgischen Nahtmaterialien aus statistischem Glykolid-TMC-Copolymer gemäß der Erfindung durch eine höhere Anfangsfestigkeit im Vergleich zu bekannten resorbierbaren multifilen Materialien aus. Gute Ergebnisse können Filamente gebildet aus 84 Gew.-% Glykolid und 16 Gew.-% Trimethylencarbonat aufweisen. Sowohl bei in vitro wie bei in vivo Versuchen zeigt sich, daß die Festigkeiten der erfindungsgemäßen Proben während der ersten drei Tage nur langsam abnehmen und dabei über den Werten der bekannten Multifilamentmaterialien bleiben. Danach zeigt sich ein beschleunigter Abbau der Festigkeiten. Besonders ausgeprägt zeigt sich dieses Abbauverhalten beim Glykolid-TMC-Copolymer mit einem Monomerenverhältnis von 92/8.

Mit Vorteil ist chirurgisches Nahtmaterial aus resorbierbarem synthetischem Polymer ausgebildet als monofiles Filament aus Glykolid-Trimethylencarbonat-Copolymer mit statistischer Verteilung der Monomeren zur Verwendung zum Wundverschluß mit beschleunigter Resorption geeignet. Die oben genannten vorteilhaften mechanischen Eigenschaften von monofilen Nähfäden aus statistischem Glykolid-TMC-Copolymer erlauben eine einfache Handhabung des Nahtmaterials während des Vernähens von Gewebe in einem tierischen oder menschlichen Körper, z. B. beim Fixieren von Organen, Schließen von Rissen im Körpergewebe oder Schließen von chirurgischen Schnitten. Insbesondere durch die Ausbildung eines Monofilaments mit glatterer Fadenoberfläche als ein multifiler Nähfaden kann das zu behandelnde Gewebe vor Schädigung durch die Nahtlegung bewahrt werden. Dadurch wird die Gefahr von für den Patienten belastenden Nebenwirkungen, wie z. B. verzögerte Heilung und Gewebegranulombildung eingeschränkt. Die gute Knüpfbarkeit und Knotenfestigkeit in Verbindung mit der hohen Anfangszugfestigkeit und Dehnbarkeit erlauben eine zuverlässige Fixierung und Stabilisierung der verbundenen Wundränder während der ersten Tage nach dem chirurgischen Eingriff. Insbesondere kann während dieser ersten Tage sich neubildendes körpereigenes Gewebe zuverlässig zur natürlichen Wundheilung dienen, da die Gefahr eines Auseinanderreissens der Wundränder bei Bewegung des Patienten durch die sichere Fixierung verringert ist.

Ein Verfahren zur Herstellung von chirurgischem Nahtmaterial aus resorbierbarem synthetischen Polymer ist dadurch gekennzeichnet, daß Glykolid- und Trimethylencarbonat-Monomer simultan zu einem statistischen Copolymer umgesetzt werden. Als Monomere können 1,4-Dioxan-2,5-dion und 1,3-Dioxan-2-on in für das gewünschte Copolymer notwendigen Anteilen eingesetzt werden. Der Monomerenmischung kann ein geeigneter Katalysator, z. B. Zinnoctoat, in der üblichen erforderlichen Menge zugesetzt werden. Die Umsetzung wird als Schmelzpolymerisation in einem geeigneten Reaktor durchgeführt, der heizbar und mit einer Rührvorrichtung versehen ist. Insbesondere muß dieser Polymerisationsreaktor so konzipiert sein, daß die entstehenden hochviskosen Schmelzen homogenisiert, die geforderten Temperaturbereiche eingehalten werden können und das Rohpolymerisat aus dem Reaktor weitgehend vollständig abgelassen werden kann. Reaktoren dieser Art werden unter anderem von den Firmen Werner & Pfleiderer in Stuttgart, AMK in Aachen oder Haake in Karlsruhe angeboten.

Die Copolymerisierungsreaktion kann nach üblichen den Fachleuten bekannten Verfahrenweisen zur Herstellung von statistischen Copolymeren durchgeführt werden. Bevorzugt kann die Reaktionsmischung unter stetiger Durchmischung erhitzt werden, insbesondere auf eine Temperatur von 170 bis 190 °C, bevorzugt von 175 bis 185 °C. Während einer Reaktionsdauer von 30 bis 100 Minuten können sich die vorgelegten Monomeren zu einem statistischen Copolymer umsetzen.

Nach Beendigung der Umsetzung wird das rohe Copolymer über die Reaktionstemperatur hinaus kurzzeitig erhitzt und als Schmelze ausgetragen und nach Erkalten in üblicher Weise granuliert. Durch Extraktion, beispielsweise mit Ethylacetat oder einem anderen geeigneten Lösemittel, können niedermolekulare Anteile und Restmonomere abgetrennt werden. Das so erhaltene Polymergranulat wird anschließend getrocknet. Das Trocknen kann nach üblichen Verfahren vorgenommen werden, insbesondere bei erhöhter Temperatur und/oder unter reduziertem Druck. Am rohen Copolymer noch anhaftende Reste von Extraktionsmittel können während des Trocknens durch Verdampfen entfernt werden. Ebenso können Reste von Monomeren gegebenenfalls während des Trocknens eliminiert werden. Gegebenenfalls kann ein weiterer Reinigungsschritt zur Erzielung eines hochreinen Copolymers gemäß der Erfindung ausgeführt werden. Bevorzugt kann eine solche Reinigung vor dem Trocknungsschritt erfolgen.

Das statistische Glykolid-TMC-Copolymer mit einem Zusammensetzungsverhältnis gemäß der Erfindung zeichnet sich aus durch einen Schmelzbereich von 170 bis 215 °C. Dieser Schmelzbereich kann insbesondere bei höherem Glykolidanteil im Bereich der höheren Temperaturen liegen. Ferner zeichnen sich die erfindungsgemäß hergestellten Glykolid-TMC-Copolymere durch eine Schmelzenthalpie im Bereich von 40 bis 65 J/g aus. Bei Copolymeren mit höherem Glykolidanteil kann die Schmelzenthalpie im Bereich der höheren Werte liegen.

Das erfindungsgemäße Copolymer ist in seiner Struktur teilkristallin. Dies ist bedingt durch den hohen Glykolidanteil von über 76 Gew.- %. Durch den hohen Glykolidgehalt wird das Polymermaterial steifer, was seine Eigenschaften beim praktischen Einsatz als chirurgisches Nahtmaterial beeinflußt. Mit Vorteil kann das Nahtmaterial einen Weichmacher enthalten. Der Weichmacher kann in Mengen von 1 - 25 Gew.-%, bezogen auf das Gesamtgewicht von Copolymer und Weichmacher, im Nahtmaterial enthalten sein. Der Weichmacher zeichnet sich dadurch aus, daß er im Copolymer physikalisch gelöst ist. Der Weichmacher ist biologisch verträglich und/oder biologisch abbaubar. Mit Vorteil können für erfindungsgemäße Glykolid-TMC-Copolymere Glycerintriacetat, Butylcitrat, Triethylcitrat oder Acetyltributylcitrat als Weichmacher verwendet werden. Vorzugsweise können Oligomere von ε-Caprolacton als Weichmacher verwendet werden. Ferner sind auch Oligomere von TMC als Weichmacher im erfindungsgemäßen Copolymer geeignet. Erfindungsgemäß verwendbare Oligomere weisen mit Vorteil ein Molgewichtsmaximum von 22000, insbesondere 20000 auf, was etwa 400 Caprolacton-Einheiten entspricht. Der Weichmacher besitzt vorzugsweise eine Viskosität im Bereich zähflüssig bis fest. Die inhärente Viskosität liegt bei 25 °C insbesondere im Bereich von 0,05 bis 0,5 dl/g. Zum Zusammenhang von Molekulargewicht und Viskosität bei Polymeren siehe Schindler, A. et al. in: Journ. Polym. Sci., Vol. 20, S. 319 - 326 (1982) und Rafler, E. in: Acta Polym. 44, S. 168 - 170 (1993). Die Vermischung des Weichmachers mit dem Copolymer kann in der Schmelze oder in Lösung erfolgen. Die zugegebene Weichmachermenge kann in etwa umgekehrt proportional zur Menge an TMC im Copolymeren sein. Das TMC im Copolymer wirkt als sogenannter innerer Weichmacher. Ein zusätzlich enthaltener Weichmacher wirkt als sogenannter äußerer Weichmacher. Das erfindungsgemäße Copolymer mit Weichmachergehalt besitzt keine elastomeren Eigenschaften.

Chirurgisches Nahtmaterial kann aus dem erfindungsgemäß hergestellten Polymermaterial nach üblichen den Fachleuten bekannten Verfahren hergestellt werden. Insbesondere kann das statistische Glykolid-TMC-Copolymer gemäß der Erfindung in einem Schmelzspinnverfahren zu Monofilamenten versponnen werden. Beispielsweise kann das erfindungsgemäße Polymermaterial in einer üblichen Extruderanlage, z. B. einem Einschneckenextruder, auf seine Schmelztemperatur erwärmt und durch geeignete Spinndüsen zu Monofilamenten extrudiert werden. In einer bevorzugten Ausführungsform kann getrocknetes Glykolid-TMC-Copolymer bei einer Temperatur im Bereich von 170 bis 235 °C aufgeschmolzen werden. Zur Extrusion kann bevorzugt eine Spinndüse mit 0,5 mm bis 1,5 mm Durchmesser verwendet werden. Mit Vorteil kann das gebildete Filament in ein Kühlbad, bevorzugt Wasser bei Raumtemperatur, extrudiert werden.

Um die erforderlichen mechanischen Eigenschaften zu erhalten, kann das extrudierte Filament zur Orientierung der Molekülketten verstreckt werden. Mit Vorteil kann es mit einem Verstreckverhältnis von 1 : 4 bis 1 : 10 verstreckt werden. Um einen andauernden Erhalt der Orientierung, der mechanischen Eigenschaften sowie der Dimensionsstabilität der Filamente zu sichern, kann das verstreckte Polymermaterial fixiert werden. Das Fixieren erfolgt bei Temperaturen im Bereich zwischen Raumtemperatur und 95 °C, bevorzugt bei 40 bis 80 °C. Besonders bevorzugt ist es, Verstrecken und Fixieren unmittelbar anschließend an die Extrusion, insbesondere in einem kombinierten Verfahren durchzuführen. Mit Vorteil kann dazu eine entsprechende Apparatur von kombinierten geeigneten Vorrichtungen verwendet werden.

Die Durchmesser der auf diese Weise hergestellte Monofilamente können im üblichen Bereich von 0,001 bis 0,8 mm liegen. Mit Vorteil sind diese Monofilamente gemäß der Erfindung ferner durch die oben genannten mechanischen Eigenschaften gekennzeichnet.

Erfindungsgemäß hergestellte Glykolid-TMC-Copolymer-Filamente können nach üblichen Methoden zu chirurgischem Nahtmaterial verarbeitet, z. B. auf geeignete Längen zugeschnitten werden. Insbesondere kann das erfindungsgemäße Polymermaterial in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Bevorzugt kann eine Sterilisierung des erfindungsgemäßen Nahtmaterials unter Verwendung von Ethylenoxid vorgenommen werden.

Mit Vorteil kann das chirurgische Nahtmaterial in zweckmäßiger Länge zugeschnitten gebrauchsfertig in geeigneter Weise verpackt vorliegen. In einer bevorzugten Ausführungsform können die erfindungsgemäßen Nähfäden mit chirurgischen Nadeln versehen vorliegen.

Wegen der hydrolytischen Zersetzbarkeit des erfindungsgemäßen Polymermaterials sind die Nahtmaterialien bei ihrer Lagerung vor Feuchtigkeit und erhöhten Temperaturen zu schützen, damit die Festigkeitseigenschaften bis zur Verwendung voll erhalten bleiben. Mit Vorteil können die gemäß der Erfindung hergestellten chirurgischen Nahtmaterialien in gebrauchsbereitem Zustand getrocknet und in geeigneter Weise verpackt werden. Zweckmäßigerweise kann dies durch eine vor Feuchtigkeit schützende Verpackung geschehen, insbesondere einer Verpackung aus feuchtigkeitsundurchlässigem Folienmaterial, bevorzugt einer Vakuumverpackung. Ferner durch Auswahl eines trockenen kühlen Lagerortes.

In einer besonders bevorzugten Ausführungsform können erfindungsgemäße chirurgische Nähfäden aus Glykolid-TMC-Copolymer gebrauchsfertig zugeschnitten, mit chirurgischen Nadeln versehen werden und sterilisiert in einer vor Feuchtigkeit schützenden und für einfache Entnahme zweckmäßigen sterilen Verpackung vorliegen.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Ferner nehmen die Beispiele Bezug auf die begleitenden Zeichnungen in denen
- Figur 1: die Veränderung der Zugfestigkeiten in vitro über eine Inkubationszeit von 14 Tagen von zwei erfindungsgemäßen Nahtmaterialien und einem Vergleichsmaterial aus dem Stand der Technik zeigt und
- Figur 2: die Veränderung der Zugfestigkeiten in vivo über eine Implantationszeit von 10 Tagen von zwei erfindungsgemäßen Nahtmaterialien und einem Vergleichsmaterial aus dem Stand der Technik zeigt.

Die Beispiele dienen lediglich der Erläuterung der vorliegenden Erfindung, die in keiner Weise darauf beschränkt sein soll.

### BEISPIEL 1 (Vergleich)

Herstellung von Glycolid-TMC-Copolymer mit einem Monomerenverhältnis von 76/24.

Die Polymerisation erfolgt in einem Helicone-2 CV Reaktor mit einem nutzbaren Volumen von ca. 200 ml. Zur Synthese des statistischen Copolymers werden 146 g Glykolid und 54 g Trimethylencarbonat unter Inertbedingungen eingewogen und mit 80 ppm Zinn (als Zinnoctoat in toluolischer Lösung) versetzt. Nach Entfernen des Toluols im Hochvakuum wird diese Mischung innerhalb von 15 bis 20 Minuten bei 100 °C aufgeschmolzen, homogenisiert und dann in den vorgeheizten Reaktor eingetragen. Die Reaktionszeit beträgt 1 Stunde bei 190 °C. Die Polymerisation erfolgt unter Schutzgas (Argon) und unter Rühren. Die Ausbeute beträgt ca. 150 g. Die Analyse der Rohprodukte ergibt eine inhärente Viskosität von 1,4 bis 1,6 dl/g (HFIP, 30 °C). Nach Zerkleinerung des Rohpolymerisates und Mahlung über einem 4 mm Sieb unter CO₂-Kühlung wird das Copolymer zur Entfernung von unumgesetzten Monomeren, Oligomeren, Katalysatoren und unerwünschten Nebenprodukten durch mehrmalige Extraktion mit Ethylacetat gereinigt und anschließend 4 Tage bei 50 °C im Vakuum getrocknet. Die inhärente Viskosität des Polymers beträgt 1,2 bis 1,5 dl/g, seine Schmelzenthalpie beträgt 30 bis 50 J/g.

### BEISPIEL 2

### Herstellung von Glycolid-TMC-Copolymer mit einem Monomerenverhältnis von 84/16

Zur Synthese des statistischen Copolymers werden 231 g 1,4-Dioxan-2,5-dion und 69 g 1,3-Dioxan-2-on unter Inertbedingungen (Stickstoff) eingewogen und mit 53,3 ppm Zinn (als Zinnoctoat in toluolischer Lösung) als Katalysator vermischt. Nach Entfernen des Toluols im Hochvakuum wird die Mischung innerhalb von 15 bis 20 Minuten bei 100 °C aufgeschmolzen, homogenisiert und dann in den vorgeheizten Reaktor (Kneter Rheomix, von Fa. Haake-Buchler) eingetragen. Die Polymerisation erfolgt bei einer Reaktionstemperatur von 170 °C (max. Massetemperatur 205 °C) über eine Reaktionszeit von 42 Minuten. Danach wird die Reaktionstemperatur für eine Zeitspanne von 8 Minuten auf 195 °C gesteigert. Anschließend wird die Reaktionsschmelze aus dem Reaktor abgelassen. Die Polymerisation erfolgt über die gesamte Reaktionszeit unter Schutzgas Argon. Die Materialausbeuten betragen 235 bis 265 g pro Ansatz. Die Rohprodukte haben eine inhärente Viskosität von 1,3 bis 1,6 dl/g (HFIP, 30 °C). Das Rohprodukt wird über einem 4 mm Siebeinsatz unter CO₂-Kühlung gemahlen und anschließend im Vakuum 3,5 Tage bei 50 °C getrocknet. Die Reinigung des Rohpolymerisates von unumgesetztem Monomeren, Oligomeren, Katalysatoren und unerwünschten Nebenprodukten erfolgt durch Extraktion mit Ethylacetat und/oder überkritischem CO₂. Das gereinigte, bei 50 °C über 3 Tage im Vakuum nachgetrocknete Copolymerisat wird dann über einem 1 mm Siebeinsatz fraktioniert und unter Feuchtigkeitsausschluß luftdicht verpackt. Die inhärente Viskosität des Polymers beträgt 1,3 bis 1,6 dl/g, seine Schmelzenthalpie beträgt 40 bis 55 J/g.

### BEISPIEL 3

### Herstellung von Glycolid-TMC-Copolymer mit einem Monomerenverhältnis von 92/8

Zur Synthese werden 261 g 1,4-Dioxan-2,5-dion und 39 g 1,3-Dioxan-2-on unter Inertbedingungen (Stickstoff) eingewogen und 53,3 ppm Zinn (als Zinnoctoat in toluolischer Lösung) als Katalysator vermischt. Nach Entfernen des Toluols im Hochvakuum wird diese Mischung innerhalb von 15 bis 20 Minuten bei 100 °c aufgeschmolzen, homogenisiert und dann in den vorgeheizten Reaktor (Kneter Rheomix, von Fa. Haake-Buchler) eingetragen. Die Polymerisation erfolgt bei einer Reaktionstemperatur von 185 °C (max. Massetemperatur 215 °C) über eine Reaktionszeit von 37 Minuten. Danach wird die Reaktionstemperatur für eine Zeitspanne von 8 Minuten auf 200 °C gesteigert. Anschließend wird die Reaktionsschmelze aus dem Reaktor abgelassen. Die Polymerisation erfolgt über die gesamte Reaktionszeit unter Schutzgas Argon. Die Ausbeuten betragen 250 bis 265 g pro Ansatz. Die Rohprodukte haben eine inhärente Viskosität von 1,3 bis 1,5 dl/g (HFIP, 30 °C). Die Aufarbeitung des Rohprodukts erfolgt gemäß Beispiel 2. Die inhärente Viskosität des Polymers beträgt 1,2 bis 1,5 dl/g, seine Schmelzenthalpie beträgt 50 bis 65 J/g.

### BEISPIEL 4

### Herstellung von Monofilamenten aus Glycolid-TMC-Copolymer

Gemäß Beispiel 3 hergestellte Glykolid-TMC-Copolymer-Chips, die 24 Stunden lang bei 50 °C im Hochvakuum auf eine Restfeuchte von weniger als 0,01 Gew.-% getrocknet wurden, werden in einen Doppelschneckenextruder eingespeist. In den vier Heizzonen wird das Polymer bei Temperaturen von 190 °C bis 230 °c aufgeschmolzen. Es wird unter Schutzgas Argon gearbeitet. Die Schneckendrehzahl beträgt 10 U/min, die Spinnkopftemperatur 190 °C. Durch eine Einlochspinndüse von 1 mm Durchmesser wird bei 40 bar in ein Wasserbad von Raumtemperatur extrudiert. Die Durchzugslänge beträgt 120 cm, die Durchzugstiefe 60 cm und der Düsenabstand 10 cm. Das koagulierte Filament wird bei Raumtemperatur und einer Galettengeschwindigkeit von 25 m/min abgezogen. Zur Herstellung eines chirurgischen Nähfadens wird der Rohfaden verstreckt in einem Verstreckofen von 600 cm Länge bei 60 °C. Die Geschwindigkeiten der beiden Verstreckgaletten mit Raumtemperatur betragen 5 m/min bzw. 35 m/min, so daß sich ein Verstreckverhältnis von 1 : 7 ergibt. Das so hergestellte Monofilament wird gleichzeitig thermofixiert. Es besitzt einen Durchmesser von 0,19 mm, eine Knotenreißkraft von 17,3 N und eine Knotendehnung von 33 %.

Ein Vergleich der mechanischen Eigenschaften von verstreckten und thermofixierten Monofilen, die aus den Glykolid-TMC-Copolymeren der Beispiele 1 bis 3 hergestellt wurden, ist in der folgenden Tabelle angegeben.

| Copolymer Glykolid/TMC | 76/24 (Vergleich) | 84/16 | 92/8 |
|---|---|---|---|
| Inhärente Viskosität Copolymer | 1,4 dl/g | 1,5 dl/g | 1,3 dl/g |
| Monofilament Durchmesser | 0,28 mm | 0,14 mm | 0,19 mm |
| Knotenreißkraft | 13,8 N | 8,7 N | 17,3 N |
| Dehnung | 111 % | 38 % | 33 % |

Das Monofil aus statistischem Copolymer mit 76 % Glykolid und 24 % TMC entspricht den Anforderungen an einen chirurgischen Nähfaden nicht.

### BEISPIEL 5

### Abbau von Glykolid-TMC-Copolymer in vitro

Zur Untersuchung des Abbauverhaltens von gemäß den Beispielen 1 bis 4 hergestellen Glykolid-TMC-Copolymer-Monofilamenten werden sterilisierte Proben bei 37 °C in Phosphatpuffer von pH 6,1 für zwei Wochen inkubiert. Vor Beginn des Versuches sowie an bestimmten Versuchstagen werden Proben entnommen und auf ihre Zugfestigkeit geprüft. Verwendet wird hierfür eine Universal-Prüfmaschine der Firma INSTRON bei einem Klemmbackenabstand von 10 cm und einer Querjochgeschwindigkeit von 30 cm/min.

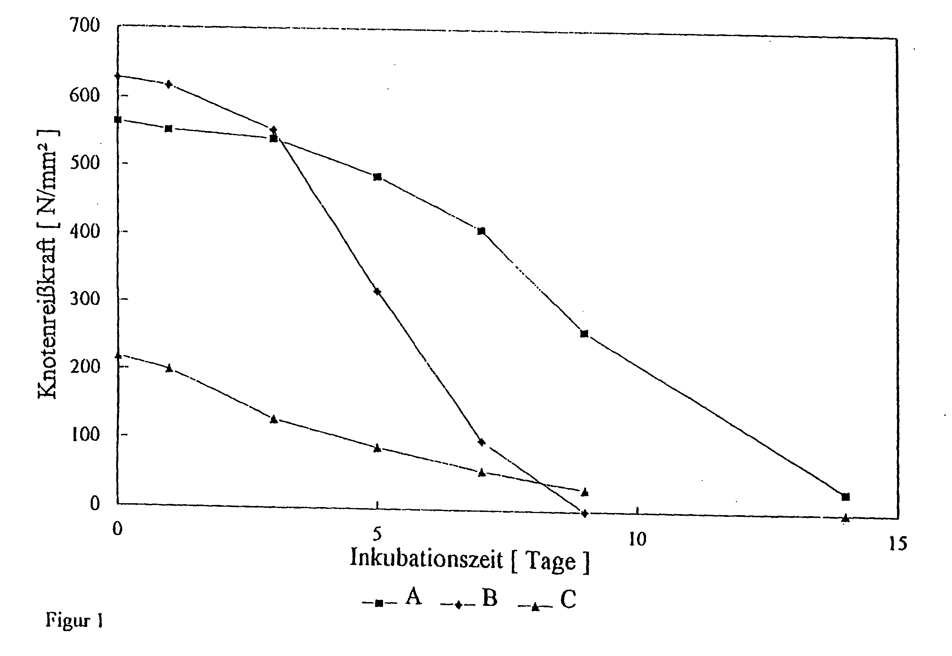

Die Ergebnisse der Zugfestigkeitsprüfungen für ein erfindungsgemäßes Nahtmaterial A mit einem Glykolid-TMC-Verhältnis von 84/16, ein erfindungsgemäßes Nahtmaterial B mit einem Glykolid-TMC-Verhältnis von 92/8 sowie ein Nahtmaterial C aus einem handelsüblichen resorbierbaren Multifilament sind in der folgenden Tabelle angegeben und in Figur 1 dargestellt.

### Zugfestigkeit (N/mm²) in vitro

| Probe | A | B | C |
|---|---|---|---|
| Verhältnis Glykolid/TMC | 84/16 | 92/8 | - |
| Inkubationszeit (Tage) | | | |
| 0 | 565 | 624 | 219 |
| 1 | 553 | 619 | 200 |
| 3 | 541 | 554 | 128 |
| 5 | 487 | 319 | 88 |
| 7 | 410 | 100 | 56 |
| 9 | 262 | 0 | 31 |
| 14 | 30 | 0 | 0 |

### BEISPIEL 6

### Abbau von Glykolid-TMC-Copolymer in vivo

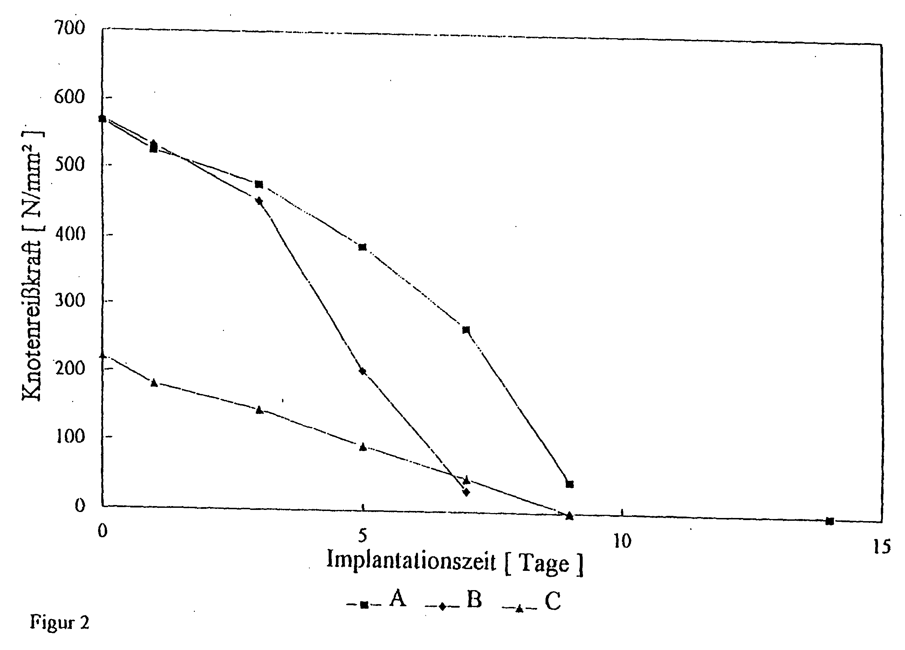

Zur Untersuchung des Abbauverhaltens und der Resorption von gemäß den Beispielen 1 bis 4 hergestellen Glykolid-TMC-Copolymer-Monofilamenten werden ethylenoxidsterilisierte Fadenbündel durch subcutane Implantation in Kaninchen eingebracht. Vor Versuchsbeginn und an bestimmten Versuchstagen werden Proben entnommen und auf ihre Zugfestigkeit (Knotenreißkraft) untersucht. Verwendet wird hierzu eine Universalprüfmaschine der Firma INSTRON bei einem Klemmbackenabstand von 10 cm und einer Querjochgeschwindigkeit von 30 cm/min. Die Ergebnisse der Zugfestigkeitsprüfungen für ein erfindungsgemäßes Nahtmaterial A mit einem Glykolid-TMC-Verhältnis von 84/16, ein erfindungsgemäßes Nahtmaterial B mit einem Glykolid-TMC-Verhältnis von 92/8 sowie ein Nahtmaterial C aus einem handelsüblichen resorbierbaren Multifilament sind in der folgenden Tabelle angegeben und in Figur 2 dargestellt.

### Zugfestigkeit (N/mm²) in vivo

| Probe | A | B | C |
|---|---|---|---|
| Verhältnis Glykolid/TMC | 84/16 | 92/8 | - |
| Inkubationszeit (Tage) | | | |
| 0 | 568 | 571 | 222 |
| 1 | 525 | 533 | 181 |
| 3 | 478 | 454 | 144 |
| 5 | 389 | 205 | 93 |
| 7 | 268 | 30 | 48 |
| 9 | 45 | 0 | 0 |
| 14 | 0 | 0 | 0 |

### Beispiel 7

### Herstellung von Monofilamenten aus Glycolid-TMC-Copolymer mit Weichmachergehalt

Beispiel 4 wurde wiederholt, wobei dem aufgeschmolzenen Copolymer im Doppelschneckenextruder in einer Schmelzzone aufgeschmolzenes oligomer von ∈-Caprolacton zudosiert wurde. Die Menge an Oligomer betrug 15 Gew.-%, bezogen auf das Gesamtgewicht von Copolymer und Oligomer. Die extrudierten monofilen Fäden zeigten im Vergleich zu Fäden ohne Weichmacher ein besseres Handling und verbesserte Knoteneigenschaften. Die günstigen Werte der Reißfestigkeit und der Abbaubarkeit waren nicht nachteilig beeinflußt.

## Patentansprüche

1. Chirurgisches Nahtmaterial aus resorbierbarem synthetischem Polymer, **dadurch gekennzeichnet, dass** es aus Glykolid-Trimethylencarbonat-Copolymer mit einem Glykolid-Gehalt grösser als 76 Gew.-% mit statistischer Verteilung der Monomeren gebildet ist und eine Bruchdehnung von 25 bis 45 % besitzt.

2. Chirurgisches Nahtmaterial nach Anspruch 1, **dadurch gekennzeichnet, dass** es als monofiles Filament, insbesondere mit einem Durchmesser von 0,001 bis 0,8 mm ausgebildet ist.

3. Chirurgisches Nahtmaterial nach Anspruch 1 oder 2, **dadurch gekennzeichnet, dass** sein Glykolid-Gehalt mehr als 78 Gew.-%, bezogen auf das gesamte Copolymer, beträgt.

4. Chirurgisches Nahtmaterial nach einem der vorhergehenden Ansprüche, **dadurch gekennzeichnet, dass** sein Glykolid-Gehalt 80 bis 99 Gew.-%, bevorzugt 83 bis 93 Gew.%, bezogen auf das gesamte Copolymer, beträgt.

5. Chirurgisches Nahtmaterial nach einem der vorhergehenden Ansprüche, **dadurch gekennzeichnet, dass** es eine Kristallinität von 15 bis 40 % aufweist.

6. Chirurgisches Nahtmaterial nach einem der vorhergehenden Ansprüche, **dadurch gekennzeichnet, dass** es eine inhärente Viskosität von 1,0 bis 2,0 dl/g, insbesondere von 1,2 bis 1,6 dl/g aufweist.

7. Chirurgisches Nahtmaterial nach einem der vorhergehenden Ansprüche, **dadurch gekennzeichnet, dass** es eine Zugfestigkeit von 250 bis 900 N/mm², insbesondere von 300 bis 650 N/mm² aufweist.

8. Chirurgisches Nahtmaterial nach einem der vorhergehenden Ansprüche, **dadurch gekennzeichnet, dass** es eine Bruchdehnung von 30 bis 40 % aufweist.

9. Chirurgisches Nahtmaterial nach einem der vorhergehenden Ansprüche, **dadurch gekennzeichnet, dass** es einen Weichmacher enthält.

10. Chirurgisches Nahtmaterial nach Anspruch 9, **dadurch gekennzeichnet, dass** der Weichmacher in Mengen von 1 - 25 Gew.-%, bezogen auf das Gesamtgewicht von Copolymer und Weichmacher, im Nahtmaterial enthalten ist.

11. Chirurgisches Nahtmaterial nach einem der Ansprüche 9 oder 10, **dadurch gekennzeichnet, dass** der Weichmacher physikalisch im Copolymer gelöst ist.

12. Chirurgisches Nahtmaterial nach einem der Ansprüche 9 bis 11, **dadurch gekennzeichnet, dass** die Menge an Weichmacher im Copolymeren umso grösser ist, je grösser der Gehalt an Glykolid im Copolymeren ist.

13. Chirurgisches Nahtmaterial nach einem der vorhergehenden Ansprüche, **dadurch gekennzeichnet, dass** es als Weichmacher Oligomere von epsilon-Caprolacton im Nahtmaterial enthält.

14. Chirurgisches Nahtmaterial nach einem der vorhergehenden Ansprüche, **dadurch gekennzeichnet, dass** es als Weichmacher Oligomere von TMC im Nahtmaterial enthält.

15. Chirurgisches Nahtmaterial nach einem der Ansprüche 14 oder 15, **dadurch gekennzeichnet, dass** es die Oligomere in Mengen von 1 - 25 Gew.-%, vorzugsweise in Mengen von 5 - 20 Gew.-%, bezogen auf das Gewicht des Nahtmaterials enthält.

16. Verwendung eines resorbierbaren synthetischen Polymers ausgebildet als monofiles Filament aus Glykolid-Trimethylencarbonat-Copolymer mit einem Glykolid-Gehalt grösser als 76 Gew.-% mit statistischer Verteilung der Monomeren und einer Bruchdehnung von 25 bis 45 %, insbesondere nach einem der vorhergehenden Ansprüche, zur Herstellung eines chirurgischen Nahtmaterials zum Wundverschluss mit beschleunigter Resorption.

## Claims

1. Surgical suture material of resorbable, synthetic polymer, **characterized in that** it is formed from glycolide-trimethylene carbonate copolymer with a glycolide content above 76 wt.% with a random distribution of the monomers and an elongation at break of 25 to 45%.

2. Surgical suture material according to claim 1, **characterized in that** it is in the form of a monofilament, particularly with a diameter of 0.001 to 0.8 mm.

3. Surgical suture material according to claim 1 or 2, **characterized in that** its glycolide content is more than 78 wt.%, based on the total copolymer.

4. Surgical suture material according to one of the preceding claims, **characterized in that** its glycolide content is 80 to 99, preferably 83 to 93 wt.%, based on the total copolymer.

5. Surgical suture material according to one of the preceding claims, **characterized in that** it has a crystallinity of 15 to 40%.

6. Surgical suture material according to one of the preceding claims, **characterized in that** it has an inherent viscosity of 1.0 to 2.0 dl/g, particularly 1.2 to 1.6 dl/g.

7. Surgical suture material according to one of the preceding claims, **characterized in that** it has a tensile strength of 250 to 900, particularly 300 to 650 N/mm².

8. Surgical suture material according to one of the preceding claims, **characterized in that** it has an elongation at break of 30 to 40%.

9. Surgical suture material according to one of the preceding claims, **characterized in that** it contains a plasticizer.

10. Surgical suture material according to claim 9, **characterized in that** the suture material contains the plasticizer in quantities of 1 to 25 wt.%, based on the total copolymer and plasticizer weight.

11. Surgical suture material according to one of the claims 9 or 10, **characterized in that** the plasticizer is physically dissolved in the copolymer.

12. Surgical suture material according to one of the claims 9 to 11, **characterized in that** the higher the glycolide content in the copolymer, the higher the plasticizer quantity in the copolymer.

13. Surgical suture material according to one of the preceding claims, **characterized in that** as plasticizers it contains oligomers of ε-caprolactone in the suture material.

14. Surgical suture material according to one of the preceding claims, **characterized in that** it contains oligomers of TMC as plasticizers in the suture material.

15. Surgical suture material according to one of the claims 14 or 15, **characterized in that** it contains oligomers in quantities of 1 to 25 wt.%, preferably 5 to 20 wt.%, based on the suture material weight.

16. Use of a resorbable, synthetic polymer in the form of a monofilament from glycolide-trimethylene carbonate copolymer with a glycolide content above 76 wt.% and with a random distribution of the monomers and an elongation at break of 25 to 45%, particularly according to one of the preceding claims, for producing a surgical suture material for wound closure with accelerated resorption.

## Revendications

1. Matériel de suture chirurgicale en polymère synthétique résorbable, **caractérisé en ce qu'**il est constitué de copolymère de glycolide et de carbonate triméthylène présentant une teneur en glycolide supérieure à 76 % en poids avec une répartition statique des monomères et disposant d'un allongement à la rupture de 25 à 45 %.

2. Matériel de suture chirurgicale selon la revendication 1, **caractérisé en ce qu'**il est constitué d'un filament monofil, en particulier d'un diamètre compris entre 0,001 et 0,8 mm.

3. Matériel de suture chirurgicale selon la revendication 1 ou 2, **caractérisé en ce que** sa teneur en glycolide s'élève à plus de 78 % en poids par rapport à l'ensemble du copolymère.

4. Matériel de suture chirurgicale selon l'une des revendications précédentes, **caractérisé en ce que** sa teneur en glycolide est comprise entre 80 et 99 % en poids de préférence entre 83 et 93 % en poids par rapport à l'ensemble du copolymère.

5. Matériel de suture chirurgicale selon l'une des revendications précédentes, **caractérisé en ce qu'**il présente une cristallinité comprise entre 15 et 40 %.

6. Matériel de suture chirurgicale selon l'une des revendications précédentes, **caractérisé en ce qu'**il présente une viscosité inhérente de 1,0 à 2,0 dl/g, en particulier de 1,2 à 1,6 dl/g.

7. Matériel de suture chirurgicale selon l'une des revendications précédentes, **caractérisé en ce qu'**il présente une résistance à la traction comprise entre 250 et 900 N/mm², en particulier entre 300 et 650 N/mm².

8. Matériel de suture chirurgicale selon l'une des revendications précédentes, **caractérisé en ce qu'**il présente un allongement à la rupture de 30 à 40 %.

9. Matériel de suture chirurgicale selon l'une des revendications précédentes, **caractérisé en ce qu'**il contient des plastifiants.

10. Matériel de suture chirurgicale selon la revendication 9, **caractérisé en ce que** le matériel de suture contient des plastifiants dans des quantités comprises entre 1 et 25 % en poids, par rapport au poids total de copolymère et de plastifiant.

11. Matériel de suture chirurgicale selon l'une des revendications 9 ou 10, **caractérisé en ce que** le plastifiant est dissous physiquement dans le copolymère.

12. Matériel de suture chirurgicale selon l'une des revendications 9 à 11, **caractérisé en ce que** la quantité de plastifiant dans le copolymère est augmente proportionnellement à la teneur de glycolide dans le copolymère.

13. Matériel de suture chirurgicale selon l'une des revendications précédentes, **caractérisé en ce qu'**il contient en tant que plastifiant des oligomères de l'epsilon-caprolactone dans le matériel de suture.

14. Matériel de suture chirurgicale selon l'une des revendications précédentes, **caractérisé en ce qu'**il contient en tant que plastifiants des oligomères de TMC (carbones cycliques) dans le matériel de suture.

15. Matériel de suture chirurgicale selon l'une des revendications 14 ou 15, **caractérisé en ce qu'**il contient des polymères dans des quantités de 1 à 25 % en poids de préférence dans des quantités de 5 à 20 % en poids par apport au poids du matériel de suture.

16. Utilisation d'un polymère synthétique résorbable en tant que filment monofil en copolymère de glycolide et de carbonate triméthylène présentant une teneur en glycolide supérieure à 76 % en poids avec une répartition statique des monomères et disposant d'un allongement à la rupture de 25 à 45 %, en particulier selon l'une des revendications précédentes pour la fabrication d'un matériau de suture et pour la fermeture de plaies à résorption accélérée.
